(19) **Europäisches Patentamt**
**European Patent Office**
**Office européen des brevets**

(11) **EP 4 218 865 B1**

(12) **EUROPEAN PATENT SPECIFICATION**

(45) Date of publication and mention
of the grant of the patent:
**23.10.2024   Bulletin 2024/43**

(51) International Patent Classification (IPC):
*A61M 11/00* (2006.01)       *A61M 15/00* (2006.01)
*B05B 17/00* (2006.01)

(21) Application number: **22153887.9**

(22) Date of filing: **28.01.2022**

(52) Cooperative Patent Classification (CPC):
**A61M 15/0085; A61M 11/005; B06B 1/0246;**
B05B 17/0646; B06B 2201/55

(54) **INHALATION THERAPY APPARATUS**

INHALATIONSTHERAPIEGERÄT

APPAREIL DE THÉRAPIE PAR INHALATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**02.08.2023   Bulletin 2023/31**

(73) Proprietor: **PARI Pharma GmbH**
**82319 Starnberg (DE)**

(72) Inventors:
• **Achtzehner, Wolfgang**
**82239 Alling (DE)**
• **Reinhart, Markus**
**86919 Utting (DE)**

(74) Representative: **Hoffmann Eitle**
**Patent- und Rechtsanwälte PartmbB**
**Arabellastraße 30**
**81925 München (DE)**

(56) References cited:
EP-A1- 3 666 315         EP-A1- 3 711 797
US-A- 5 415 161          US-A1- 2005 034 719
US-A1- 2005 056 274      US-A1- 2019 321 570

## Description

## Technical Field

The invention relates to an inhalation therapy apparatus having an aerosol generator, in particular with an oscillatable membrane for nebulising a liquid or powder.

## Background

The control of inhalation therapy apparatuses occurs in many cases under consideration of the respiration of the patient so as to only generate the aerosol during inhalation or to provide the aerosol for inhalation, that is a synchronization of aerosol generation with an inhalation phase of a patient or user. In this manner, an improved application of the medicament contained in the aerosol is achieved together with a reduction in medicament losses. It is necessary for this manner of controlling inhalation apparatuses to reliably detect the respiration of the patient during inhalation therapy. Respiration sensors may be used for this purpose in conventional inhalation nebulisers, which detect the pressure fluctuations caused by the respiration of the patient and convert them into an output signal which is supplied to a control unit of the inhalation nebuliser. The output signal may be evaluated in the control unit and may be converted into suitable control processes.

EP 1 304 131 B1 shows a solution in which the control of an inhalation therapy apparatus can occur subject to the respiration of the patient without providing an additional respiration sensor. The manufacturing costs of the inhalation nebuliser is thereby reduced and the breakdown susceptibility thereof is decreased.

According to EP 1 304 131 B1, this is achieved using an inhalation therapy apparatus comprising an oscillatable membrane for nebulising a liquid, an oscillation generating device which has at least one connecting element for supplying an activation signal and by means of which the membrane is caused to oscillate when the activation signal is supplied such that a liquid disposed on one side of the membrane is nebulised through the membrane and is present on the other side of the membrane as an aerosol, and comprising a control unit from which an activation signal can be supplied to the at least one connecting unit of the oscillation generating device such that the oscillation generating device causes the membrane to oscillate, whereby an output signal generated by the respiration of the patient at the at least one connecting elements of the oscillation generating device is supplied to the control unit for controlling the inhalation therapy apparatus.

In EP 1 304 131 B1 the electromechanical transducer unit which is used to cause oscillation and which may be a piezoelectric component also provides a respiration-dependent output signal which can be used to control the inhalation therapy apparatus. Accordingly, a separate respiration sensor is no longer necessary. The reason for this is that the output signal of the oscillation generation device (e.g. an aerosol generator) includes signal components (e.g. frequency components) resulting from the respiration of the patient which can be used to control activation of the aerosol generator in synchronization with the respiration of the patient or user.

US 2019/321570 A1 teaches systems and methods of aerosol delivery with airflow regulation, whereas an example aerosol delivery device includes a mouthpiece having an airflow outlet, and an airflow passage extending between an airflow inlet and the airflow outlet. The example aerosol delivery device further includes a housing configured to receive a cartridge that includes an aerosolizable substance and a vapor element configured to heat the aerosolizable substance, and an internal power source configured to provide electrical power. The example aerosol delivery device further includes a controller coupled to the internal power source to receive a portion of the electrical power and configured to, when the cartridge is installed at the housing, cause the vapor element of the cartridge to heat the aerosolizable substance to release an aerosol into the airflow passage during an inhalation through the airflow outlet, and a connector configured to receive power from an external source to recharge the internal power source.

## Summary

## Technical Problem

According to the conventional solution described above, the output signal of the oscillation generation device has to be processed to filter out high-frequency (e.g. having a frequency in the order of 100 kHz or above) components of the driving signal (e.g. having a voltage in the order of 100V) of the oscillation generation device and to amplify the filtered signal to thus detect the weaker (e.g. having a voltage in the order of uV - mV) and low-frequency (e.g. having a frequency in the order of 0.1 - 1 Hz) components related to breathing of the user or patient.

The conventional solution may, however, not provide an accurate detection of the weaker low-frequency components related to breathing of the user or patient. The present inventors have realized that inaccuracies in detection processing are caused by an instable signal that is output after the output signal of the oscillation generation device has been filtered by a low-pass filter and amplified by an operation amplifier. Such inaccuracies impede an appropriate detection of the breathing pattern of the user and thus limit the desired accurate synchronization between the activation and deactivation of the aerosol generation with the breathing cycle of the patient or user.

In particular, the present inventors have realized that the signal that is observed after the output signal of the oscillation generation device has been filtered by a low-pass filter and amplified by an operation amplifier may have, in addition to low-frequency frequency components (e.g. having a frequency in the order of 0.1 - 1 Hz) related to breathing of the user or patient as described, additional drift components in the amplitude and is therefore not stable enough which prevents an appropriate evaluation.

The present inventors have further realized that such stability problems may be caused by an instable signal, a temperature effect, power variations, power regulations, electro-static disturbances, electro-static discharges, changes in the drive signal of the aerosol generator, and mechanical influences to the aerosol generator.

That is, electrical operating the oscillation generating device, such as an electromechanical transducer unit, in particular a piezoelectric element, generates heat and thus influences the amplitude of the output signal. Pressure fluctuations at the oscillation generating device due to a patient or user breathing in and out a nebulizing chamber further leads to variations in a cooling off effect of air flowing by the oscillation generating device and thus variations in the amount of Joule heat that can be transferred to the air flow. In other words, pressure changes at the oscillation generating device also lead to temperature variations of the oscillation generating device which have an additional effect on the output signal, in particular additional drift components, and thus prevent a stable output signal so that the breathing pattern cannot be determined accurately.

It is therefore an object of the invention to provide a more accurate detection of the breathing pattern of the user or patient from the output signal of the oscillation generation device.

## Solution

According to the invention, an inhalation therapy apparatus having an aerosol generator comprises: an oscillatable membrane for nebulising a liquid, an oscillation generating device having at least one connecting element for supplying an activation signal and by which said membrane is caused to oscillate when the activation signal is supplied such that a liquid disposed on one side of said membrane is nebulised through the membrane and is present on the other side of said membrane as an aerosol, a control unit, from which the activation signal can be supplied to the at least one connecting element of the oscillation generating device so that said oscillation generating device causes the membrane to oscillate, and a processing unit configured to clamp an output signal caused by the respiration of a patient at the at least one connecting element of the oscillation generating device.

By appropriately clamping the output signal caused by the respiration of a patient at the at least one connecting element of the oscillation generating device, the output signal can be held in a stable manner at an amplitude value around which the output signal has inhalation-induced variations but in which additional drift components of the amplitude due to the temperature effect described above are suppressed.

## Brief description of the drawings

The invention will be explained in more detail below by means of embodiments and referring to the figures:

FIG. 1 shows a schematic representation of an inhalation therapy apparatus according to the invention; and

FIG. 2 shows a block diagram of the processing unit of the inhalation therapy apparatus shown in **FIG. 1** according to an embodiment.

FIG. 3 shows another block diagram of the processing unit of the inhalation therapy apparatus shown in **FIG. 1** according to another embodiment.

FIG. 4 shows another block diagram of the processing unit of the inhalation therapy apparatus shown in **FIG. 1** according to another embodiment.

Fig. 5 shows a schematic illustration of a comparison between a non-clamped respiration signal and a clamped respiration signal.

Fig. 6 shows another schematic illustration of a comparison between a non-clamped respiration signal and a clamped respiration signal.

## Detailed description

Example embodiments of the invention are now described in detail with reference to the accompanying figures. It is noted that the following description contains examples only and should not be construed as limiting the invention. In the following, similar or same reference signs indicate similar or same elements or functions.

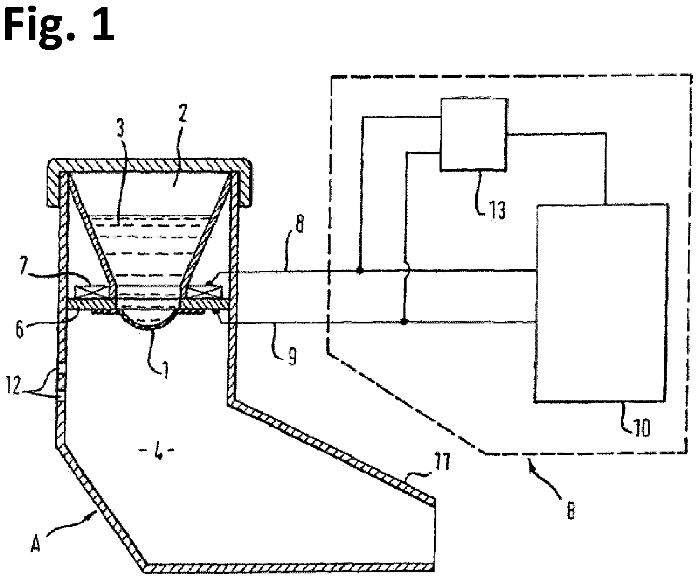

FIG. 1 shows an embodiment of an inhalation therapy apparatus having an aerosol generator, in which in a nebuliser unit A, a liquid 3 stored in a liquid reservoir 2 is nebulised by means of a membrane 1 into a nebulisation chamber 4. Nebulisation (i.e. generation of an aerosol) occurs when the membrane 1 is caused to oscillate. For this purpose, the membrane 1 may be attached to an oscillation generation device, e.g. a support unit 6 which supports the membrane 1 and to which an electromechanical transducer unit 7, for example a piezoelectric element, may be attached. The membrane 1, the support unit 6 and the electromechanical transducer unit 7 may be considered as forming the aerosol generator and may be configured in a rotationally symmetrical manner in the embodiment described herein and together form an oscillatable structure. An activation signal of a control unit 10 can be supplied to the electromechanical transducer unit 7 via connecting lines or elements 8 and 9, where the control unit 10 may be accommodated in a separate controller B in the embodiment described herein.

When the activation signal is supplied, the oscillatable structure 1, 6, 7 is caused to oscillate and the liquid 3 is nebulised through the membrane 1 and is present on the other side of the membrane as an aerosol.

A patient can inhale the aerosol provided in the nebulisation chamber 4 at the mouthpiece 11 of the nebuliser. In order to provide a sufficient supply of air, one or more air holes 12 may be provided in the housing of the nebuliser, through which ambient air can enter into the chamber 4 during inhalation and out of which the air inhaled by the patient can exit from the chamber 4 during exhalation. In comparison with the environment outside of the chamber 4, pressure fluctuations occur in the chamber 4 during inhalation and exhalation. During inhalation the pressure in the nebuliser chamber 4 sinks below the ambient pressure and during exhalation it rises above the ambient pressure.

Although pressure equalisation occurs due to the air holes 12, it has been found that pressure fluctuations are generally sufficient to act upon the membrane 1 in a way that a usable respiration-dependent output signal is emitted by (e.g. can be extracted from the output of) the electromechanical transducer unit 7. The extent of the pressure fluctuations and thus the intensity of the effect of respiration of the patient upon the membrane 1 can be influenced by the design of the air holes 12 and preferably also by correspondingly designed valve elements at the air holes 12. This valve element may be designed at least as one one-way valve for the airflow in the nebulizer chamber 4.

The nebulizer chamber 4 may further have an air outlet at the mouthpiece 11 of the nebuliser. The air outlet can be influenced by the design of the air outlet and preferably also by correspondingly designed second valve elements at the air outlet. This second valve element may be designed as a one-way valve for the airflow out of the nebulizer chamber 4.

The nebulizer chamber may contain a one-way valve for the airflow in the nebulizer chamber and a one-way valve for the airflow out of the nebulizer chamber. Therefor the generated aerosol in the nebulizer chamber may accumulate during the exhalation of the patient and a quasi-aerosol-bolus be available during the inhalation of the patient. The concept of an aerosol-bolus is mentioned in the patent application EP 00973900.4 published and granted as EP 1227856, which is included entirely with the specification and the figures. (Equivalent to US 6,962,151 and US 10/129,498)

It has been further found by tests leading to the invention described herein that the breathing-induced pressure fluctuations, as described, also lead to a temperature effect at the electromechanical transducer unit 7. In particular, electrical operating the oscillation generating device, such as the electromechanical transducer unit (e.g. a piezoelectric element), generates Joule heat and thus influences the amplitude of the output signal. While the influence of generated Joule heat on the amplitude of the output signal is known in the context of detecting the presence of a liquid on the membrane, see EP 1 558 315 A1, for example, the present inventors have realized that the breathing-induced pressure fluctuations also have a temperature effect on the operation of the electromechanical transducer unit which influences the output signal. In particular, pressure fluctuations at the oscillation generating device due to a patient or user breathing in and out a nebulizing chamber further leads to variations in a cooling off effect of air flowing by the electromechanical transducer and thus variations in the amount of Joule heat that can be momentarily transferred to the air flow. In other words, breathing-induced pressure changes at the oscillation generating device also lead to temperature variations of the oscillation generating device which have an additional effect on the output signal, in particular additional drift components, and thus prevent a stable output signal.

That is, if a normal breathing pattern of a user or patient would be considered as a periodic function in the form of, for example:

$$A_0 \sin (\omega t + \varphi_1) \qquad (1)$$

having an breathing amplitude $A_0$, breathing frequency $\omega$ (e.g. having a frequency in the order of 0.1 - 1 Hz), time t and arbitrary phase $\varphi_1$, then breathing-induced pressure changes at the electromechanical transducer unit, as explained above, may lead to a corresponding periodic output signal:

$$B_0 \sin (\omega t + \varphi_2) \qquad (2)$$

having an amplitude $B_0$ (e.g. a voltage signal in the order of uV - mV), as described above, corresponding frequency $\omega$, time t and phase $\varphi_2$ (delayed as compared to $\varphi_1$). This example is used for illustration purposes, and the skilled person understands that actual breathing pattern of a user or patient may be more complex. In any event, the present inventors have recognized that the amplitude $B_0$ of the periodic output signal is not stable but has additional drift components which prevent an accurate evaluation of the output signal to detect the breathing pattern of the user or patient. As explained, these additional drift components are due to the additional temperature effect that the air flow fluctuations have on the electromechanical transducer unit. This additional temperature effect occurs on a time scale that is larger than an inhalation or exhalation and thus leads to the observed additional drift components in the output signal.

In the shown embodiment, an output signal caused by the respiration of the patient is available at the two connecting lines/elements 8 and 9 which can also be used to supply the activation signal to the oscillatable structure 1, 6, 7. Since excitation of the membrane 1 is caused by an electric signal whose frequency is much higher than the respiration frequency of a person, an output signal, which results from the pressure fluctuations in the nebulisation chamber 4 caused by the respiration of the patient, can be picked-up at the connection lines/elements 8, 9 of the oscillatable structure 1, 6, 7. The present inventors have further realized that this output signal should be suitably clamped in a processing unit 13. This clamping of the output signal (clamped respiration signal) in the processing unit 13 provides an improved holding or stabilization of the output signal at an amplitude value ($B_0$) around which the output signal has inhalation-induced variations, i.e. suppresses additional drift components of the amplitude. This clamped respiration signal may subsequently be supplied to the control unit 10 and may be taken into account therein when activating the inhalation therapy apparatus. Here, the processing unit 13, via which the output signal at the at least one connecting lines/elements 8, 9 of the oscillation generating device 6, 7 is supplied to the control unit 10, provides a clamped respiration signal which follows the respiration process of the user or patient without additional drift terms.

As shown in the embodiment described herein, a suitable processing unit 13 is provided, which may be configured as a separate unit from the control unit 10. Alternatively, the processing unit 13 may also be integrated into the control unit 10. The processing unit 13 processes the output signal, which is influenced by the respiration of the patient, at the connections of the oscillatable structure 1, 6, 7, i.e. the aerosol generator, into a control signal (clamped respiration signal) which follows the respiration cycle of the patient. This processing may be implemented in different ways.

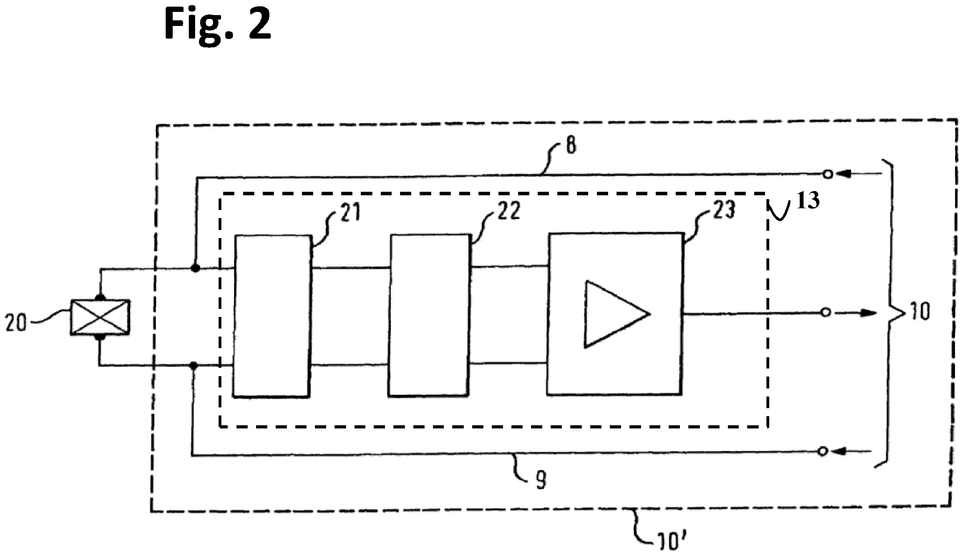

**Fig. 2** shows a preferred embodiment according to which the processing unit 13 comprises a plurality of sub-units described below. To simplify the representation in **FIG. 2,** the oscillatable structure, consisting of the membrane 1 and the oscillation generating device (e.g. the support element 6 and the piezo element 7) has been replaced in **FIG. 2** by a schematic representation of an electromechanical piezo transducer 20. The respiration-influenced output signal of the piezo transducer 20, picked-up at the connecting lines/element, is supplied in the processing unit 13 to a low-pass unit 21 which initially filters out the high frequency components of the picked-up signal, e.g. frequency components above 10 kHz, and thus, in particular, the components of the picked-up signal caused by the activation signal. The output signal of the low-pass filter 21 is supplied to a subsequent high-pass filter 22. The high-pass filter 22 may filter-out frequency components below 0.1 Hz, preferably below 0.05 Hz, more preferably below 0.01 Hz which have been found by the inventors to be related to the temperature effect at the electromechanical piezo transducer which follows the breathing-induced pressure fluctuations.

In effect, a band-pass filter is implemented for also suppressing the temperature-induced fluctuations. The output signal of the high-pass filter 22 thus becomes free of additional drift components described above and is therefore clamped or hold/stabilized at an amplitude value that results from the breathing-induced pressure fluctuations. This output signal may then be supplied to an amplifier unit 23 which converts the supplied signal into a suitable clamped output signal (clamped respiration signal) for the control unit 10. As indicated by arrows in **FIG. 2,** the clamped respiration signal processed by the processing unit 13 may be supplied to the control unit 10, from which the activation signal for the piezo transducer 20 is supplied via the connecting lines 8 and 9. The dashed line in **FIG. 2** indicates that the control unit 10 and the processing unit 13 can be arranged in a common housing or even in a single circuit arrangement/circuit.

The skilled person understands that the processing unit 13 may alternatively provide an order in which the high-pass filter 22 is followed by the low-pass filter 21 followed by the amplifier unit 23.

The skilled person further understands that instead of the amplifier unit 23, the processing unit 23 may alternatively use analogue-to-digital conversion using a high-resolution analogue-to-digital converter (ADC) having a resolution of at least 20 Bit, and applying a signal evaluation by a digital evaluation unit or a digital signal processor (DSP) having a corresponding (high) sampling rate. The digital evaluation unit or the DSP may then be coupled to the control unit to appropriately control the operation of the inhalation therapy apparatus.

The skilled person understands that the processing unit 13 may alternatively provide a band-pass filter 21' and the amplifier unit 23, wherein the band-pass filter unit 21a provides a band-pass between 0.1 Hz and at least 10 Hz, preferably 0.02 to 40 Hz to filter out both the high frequency components of the picked-up signal as well as the low frequency components due to the temperature fluctuations at the electromechanical piezo transducer.

The present inventors have further realized that lowering the cut-off frequency of the low-pass filter unit or the upper cut-off frequency of the band-pass filter to values below 10 Hz, for example 2 Hz, would lead to a situation in which the output signal (clamped respiration signal) of the processing unit 13 could not follow a switch-on or switch-off of the aerosol generator fast enough. That is, the band-pass window preferably has a sufficient size to appropriately follow the transient switch-on or switch-off behaviour.

Advantageously, the clamped respiration signal is tracked over a time period including one or more switch-on and switch-off operations to detect the presence of a fluid or liquid at the membrane. This appropriate and timely tracking of the output signal following a switch-on or switch-off is of particular advantage in cases which the operation of the aerosol generator is interrupted regularly (e.g. every 20 seconds) for detecting the presence of a fluid/liquid at the membrane, or during a power adjustment of the aerosol generator. This allows a suitable fast tracking of the clamped respiration signal over significant time periods.

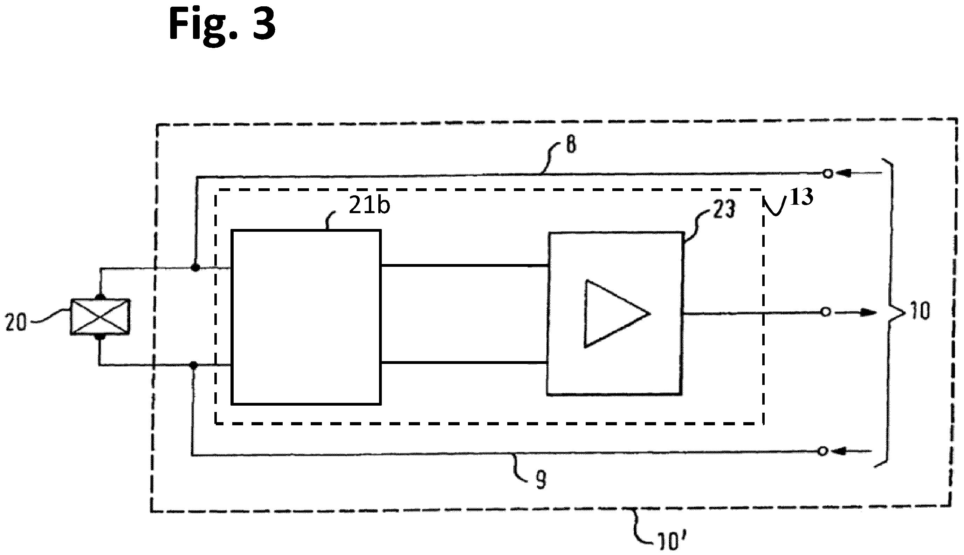

**Fig. 3** shows another preferred embodiment according to which the processing unit 13 comprises a clamped low-pass filter unit 21b and an amplifier unit 23. The clamped low-pass filter unit 21b may be a low-pass filter unit having an additional clamped circuit. The clamped circuit, e.g. a diode-based clamp (having a diode, a resistor, and a capacitor) allows to hold or clamp the output value of the low-pass filter unit at specific amplitude value. The skilled person understands that the specific amplitude value may be a value that is shifted from the DC component of the output signal caused by the respiration of a patient at the at least one connecting elements/lines 8, 9 of the oscillation generating device 6, 7, e.g. shifted from the amplitude value $B_0$ described above. This shift of the amplitude value does not negatively influence the subsequent evaluation in the control unit to properly detect and track the breathing cycle. As the resistor and capacitor of a diode-based clamp circuit determines a time constant and therefore a range of frequencies over which the clamped circuit becomes effective, the resistor and capacitor of the diode-based clamp circuit are suitably selected according to the appropriate band pass described above.

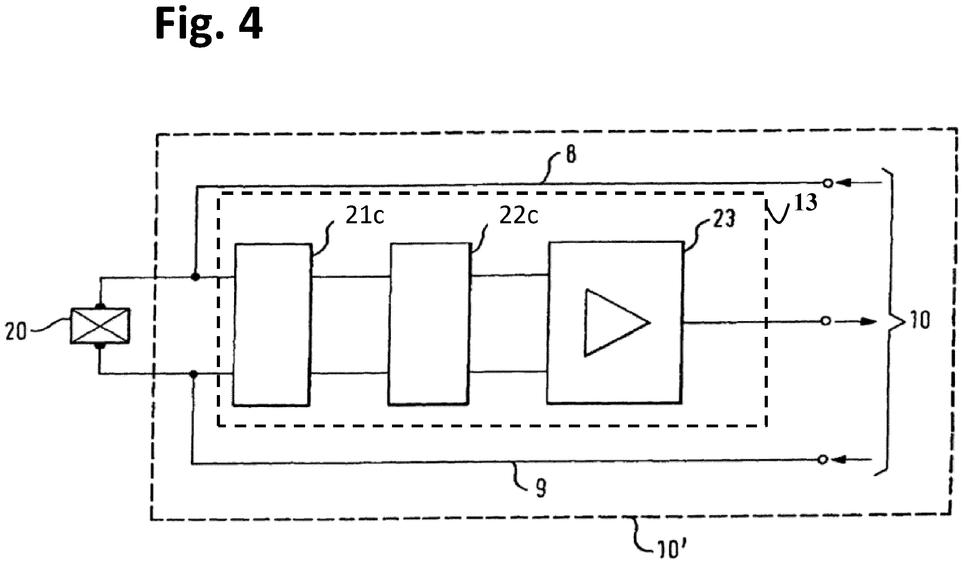

**Fig. 4** shows another preferred embodiment according to which the processing unit 13 comprises a clamped low-pass filter unit 21c, a clamped high-pass filter unit 22c and an amplifier unit 23. Here, an additional clamped circuit may be provided for both the low-pass filter and the high-pass filter, or separate clamped circuits may be provided for each of the low-pass filter and the high-pass filter. Providing separate clamped circuits may have the advantage that respective resistor and capacitor dimensions of diode-based clamp circuits may be individually selected to more appropriately and selectively address lower and upper frequency values of the appropriate band pass described above.

This clamped output signal of the processing unit 13 may subsequently be used by the control unit 10 such that the activation of the oscillatable structure 1, 6, 7 and thus nebulisation of the liquid 3 through the membrane 1 only occurs in the desired sections of the respiration cycle, for example only during inhalation.

In this manner, a more accurate on/off switching operation can be realised according to the invention, i.e. nebulisation starts when inhalation begins and ends once inhalation is complete. However, it was established during the tests based on the invention that the respiration signal of the oscillatable structure 1, 6, 7, which is usable according to the invention, allows for considerably more accurate information regarding the respiratory cycle of the patient, namely even if the activation signal is also supplied to the oscillatable structure 1, 6, 7 via the connecting lines 8 and 9. In other words, the two signals, namely the activation signal of the control unit 10, by means of which the oscillatable structure 1, 6, 7 is caused to oscillate, and the output signal of the oscillatable structure 1, 6, 7, caused by the respiration of the patient, superpose one another. However, the output signal at the connecting element 8, 9 of the oscillatable structure 1, 6, 7, which is caused by pressure fluctuations, is much smaller than the activation signal which is fed from the control unit 10 to the oscillatable structure 1, 6, 7 via the connecting lines 8 and 9, has additional drift components due to a temperature effect at the oscillatable structure and therefore requires appropriate processing in order to more reliably detect the breathing cycle.

Therefore, when using the invention, optical or acoustical indications can be given more reliably to the patient to optimise respiratory flow, or aerosol generation can be automatically shut-off more reliably if too high or too low respiratory flows are detected which are unfavourable for a safe intrapulmonary aerosol deposition.

In order to ensure a more reliable separation of the respiration signal and the activation signal, the control unit 10 is preferably configured such that no low-frequency signal components due to temperature fluctuations at the oscillatable structure 1, 6, 7 are used for nebulising the liquid 3. It is also achieved by means of this design that the output signal caused by the respiration of the patient can be more reliably separated from the activation signal with comparatively little more effort and thus in an inexpensive manner.

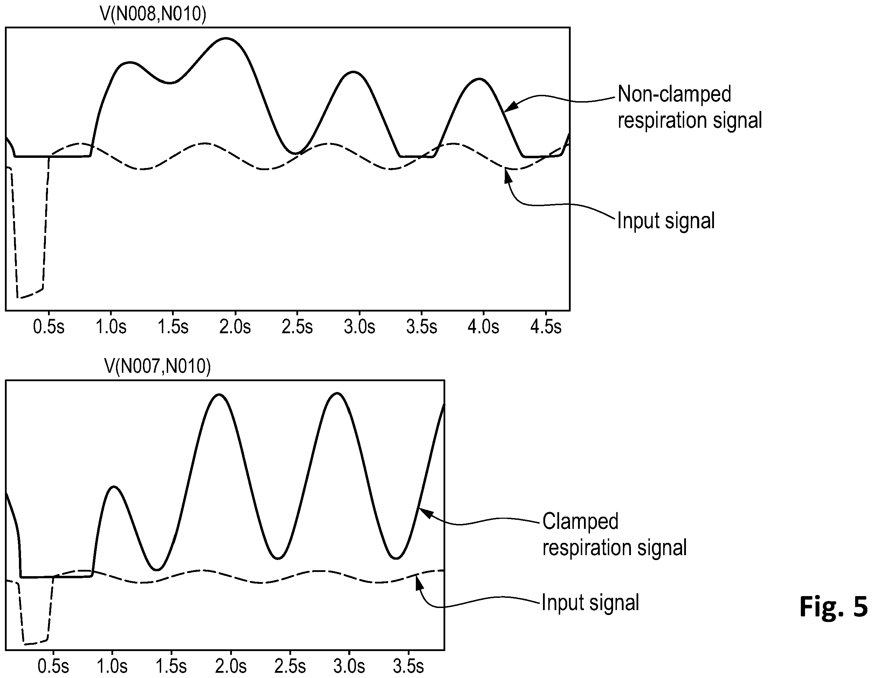

**Fig. 5** shows a schematic illustration of a comparison between a non-clamped respiration signal (upper panel) and a clamed respiration signal (lower panel). Here, in the upper panel, the X axis shows a time interval between 0.0 and 4.5 seconds while the Y-axis shows a voltage of the output signal, as well as a (dashed line) input signal having a disruption between 0.25 and 0.5 seconds and a (continuous line) output signal that is not clamped. Here, in the lower panel, the X axis shows a time interval between 0.0 and 3.5 seconds while the Y-axis shows a voltage of the output signal, as well as a (dashed line) input signal having the disruption between 0.25 and 0.5 seconds and a (continuous line) output signal that is clamped according to the embodiments of the present inventions. While the non-clamped output signal is not stable even after 4.5 seconds having outliers (drift terms) and even cutting offs, the clamped output signal is stable 1 second after the disruption and is a clamped output signal without outliers (drift terms) and cutting offs. That is, the breathing pattern can be detected accurately and fast.

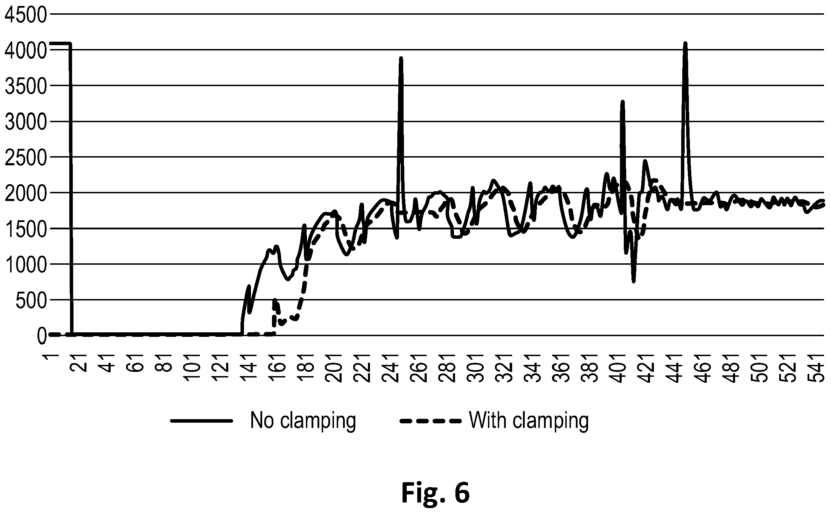

**Fig. 6** shows another schematic illustration of a comparison between a non-clamped respiration signal and a clamped respiration signal. Here, the X-axis illustrates a time axis in units of 0.1 sec (i.e. time step 541 refers to 54.1 seconds) and the Y-axis illustrates a voltage signal, in this illustrated example a digital value of

the voltage (μV) wherein the AD converter has a 12 bit resolution (with 4096 data values). The skilled person understands that the clamped output signal is free of the power peaks or interruptions (e.g. due to electrostatic discharges), e.g. at time steps 241, 401 and 441. The breathing pattern can be detected more accurately, even at the time step 401 having a large peak in the non-clamped output signal. The clamped output signal also clearly indicates that breathing has stopped after time step 441.

The control unit (10) may use the clamped output signal for a different subsequent processing, monitoring or actions, like for example a stand-by function, a pause-function, to start the activation signal, to stop the activation signal, or to show on a monitor or a display, like for example on the inhalation therapy apparatus, on a web-portal or a smart-phone in an app. The concept of a pause-function is mentioned in the patent application EP 15810730.0 published and granted as EP 3237047 which is included entirely with the specification and the figures. (Equivalent to US 2017/0368282 and US 15/535,889)

The embodiments described in more detail above show how the use according to the invention of the respiration signal occurs in an inhalation therapy apparatus having a membrane nebuliser. The description of the embodiment, however, also makes it clear that the invention can be applied to all inhalation therapy apparatuses in which an aerosol generating device is supplied with an activation signal in order to generate an aerosol, and in which the respiration of the patient acts upon the aerosol generating device such that an output signal is available which can be supplied to the control unit to activate the aerosol generating device.

The invention can be primarily used in such inhalation therapy apparatuses in which an electromechanical transducer unit is provided, which causes an oscillatable structure that effects generation of the aerosol to begin to oscillate. Inhalation therapy apparatuses in which the electromechanical transducer unit is configured in the form of a piezoelectric element are particularly suitable for the use of the invention.

The invention is defined in independent claim 1. Preferred embodiments are matter of the appended dependent claims.

## Claims

1. Inhalation therapy apparatus having an aerosol generator, comprising

   - an oscillatable membrane (1) for nebulising a liquid (3),
   - an oscillation generating device (6, 7) having at least one connecting element (8, 9) for supplying an activation signal and by which said membrane (1) is caused to oscillate when the activation signal is supplied such that a liquid disposed on one side of said membrane is nebulised through the membrane and is present on the other side of said membrane as an aerosol,
   - a control unit (10), from which the activation signal can be supplied to the at least one connecting element (8, 9) of the oscillation generating device (6, 7) so that said oscillation generating device (6, 7) causes the membrane (1) to oscillate, and
   - a processing unit (13) configured to clamp an output signal caused by the respiration of a patient at the at least one connecting element (8, 9) of the oscillation generating device (6, 7).

2. Inhalation therapy apparatus of claim 1, wherein the clamped output signal is used by the control unit (10) to control the operation of the inhalation therapy apparatus.

3. Inhalation therapy apparatus according to one of claims 1 - 2, wherein the processing unit (13) comprises a low-pass filter unit (21), a high-pass filter unit (22), and an amplifier unit (23).

4. Inhalation therapy apparatus according to one of claims 1 - 2, wherein the processing unit (13) comprises a low-pass filter unit (21), a high-pass filter unit (22) and an analogue-to-digital converter to convert the clamped output signal.

5. Inhalation therapy apparatus according to claim 4, wherein the analogue-to-digital converter is connected to a digital evaluation unit to evaluate the digitized clamped output signal.

6. Inhalation therapy apparatus according to any of claims 3 - 5, wherein the high-pass filter unit (22) filters out frequency components below 0.1 Hz, preferably below 0.05 Hz.

7. Inhalation therapy apparatus according to one of claims 1 - 2, wherein the processing unit (13) comprises a band-pass filter unit (21a) and an amplifier unit (23), wherein the band-pass filter unit (21a) provides a band-pass between 0.1 Hz and at least 10 Hz, preferably between 0.1 Hz and at least 40 Hz.

8. Inhalation therapy apparatus according to one of claims 1 - 2, wherein the processing unit (13) comprises a clamped low-pass filter unit (21b) and an amplifier unit (23) .

9. Inhalation therapy apparatus according to one of claims 1 - 2, wherein the processing unit (13) comprises a clamped low-pass filter unit (21c), a clamped high-pass filter unit (22c) and an amplifier unit (23).

**10.** Inhalation therapy apparatus according to one of claims 1 - 9, wherein the oscillation generating device (6, 7) comprises an electromechanical transducer unit (7), in particular a piezoelectric element.

**11.** Inhalation therapy apparatus according to claim 10, wherein the oscillation generating device (6, 7) comprises a support unit (6) to which the electromechanical transducer unit (7) and the membrane (1) are attached.

**12.** Inhalation therapy apparatus according to one of claims 1 - 11, wherein the processing unit (13) is integrated in the control unit (10).

**13.** Inhalation therapy apparatus according to one of claims 1 - 12, wherein an energy supply unit for the inhalation therapy apparatus is integrated in the control unit (10).


**Patentansprüche**

**1.** Inhalationstherapiegerät, die einen Aerosolgenerator aufweist, umfassend

 - eine oszillierbare Membran (1) zum Zerstäuben einer Flüssigkeit (3),
 - eine Oszillationserzeugungsvorrichtung (6, 7), die mindestens ein Verbindungselement (8, 9) zum Zuführen eines Aktivierungssignals aufweist, und durch das die Membran (1) bei Zuführung des Aktivierungssignals in Oszillierung versetzt wird, so dass eine Flüssigkeit, die sich auf einer Seite der Membran befindet, durch die Membran zerstäubt wird und auf der anderen Seite der Membran als Aerosol vorhanden ist,
 - eine Steuereinheit (10), von der aus das Aktivierungssignal dem mindestens einen Verbindungselement (8, 9) der Oszillationserzeugungsvorrichtung (6, 7) zugeführt werden kann, so dass die Oszillationserzeugungsvorrichtung (6, 7) die Membran (1) zum Oszillieren bringt, und
 - eine Verarbeitungseinheit (13), die so konfiguriert ist, dass sie ein durch die Atmung eines Patienten verursachtes Ausgangssignal an dem mindestens einen Verbindungselement (8, 9) der Oszillationserzeugungsvorrichtung (6, 7) hält.

**2.** Inhalationstherapiegerät nach Anspruch 1, wobei das gehaltene Ausgangssignal von der Steuereinheit (10) verwendet wird, um den Betrieb des Inhalationstherapiegeräts zu steuern.

**3.** Inhalationstherapiegerät nach einem der Ansprüche 1 - 2, wobei die Verarbeitungseinheit (13) eine Tiefpassfiltereinheit (21), eine Hochpassfiltereinheit (22) und eine Verstärkereinheit (23) umfasst.

**4.** Inhalationstherapiegerät nach einem der Ansprüche 1 - 2, wobei die Verarbeitungseinheit (13) eine Tiefpassfiltereinheit (21), eine Hochpassfiltereinheit (22) und einen Analog-Digital-Wandler zum Umwandeln des geklemmten Ausgangssignals umfasst.

**5.** Inhalationstherapiegerät nach Anspruch 4, wobei der Analog-Digital-Wandler mit einer digitalen Auswerteeinheit verbunden ist, um das digitalisierte gehaltene Ausgangssignal auszuwerten.

**6.** Inhalationstherapiegerät nach einem der Ansprüche 3 - 5, wobei die Hochpassfiltereinheit (22) Frequenzkomponenten unter 0,1 Hz, vorzugsweise unter 0,05 Hz, herausfiltert.

**7.** Inhalationstherapiegerät nach einem der Ansprüche 1 - 2, wobei die Verarbeitungseinheit (13) eine Bandpassfiltereinheit (21a) und eine Verstärkereinheit (23) umfasst, wobei die Bandpassfiltereinheit (21a) stellt einen Bandpass zwischen 0,1 Hz und mindestens 10 Hz, vorzugsweise zwischen 0,1 Hz und mindestens 40 Hz, bereit.

**8.** Inhalationstherapiegerät nach einem der Ansprüche 1 - 2, wobei die Verarbeitungseinheit (13) eine gehaltene Tiefpassfiltereinheit (21b) und eine Verstärkereinheit (23) umfasst.

**9.** Inhalationstherapiegerät nach einem der Ansprüche 1 - 2, wobei die Verarbeitungseinheit (13) eine gehaltene Tiefpassfiltereinheit (21c), eine gehaltene Hochpassfiltereinheit (22c) und eine Verstärkereinheit (23) umfasst.

**10.** Inhalationstherapiegerät nach einem der Ansprüche 1 - 9, wobei die Oszillationserzeugungsvorrichtung (6, 7) eine elektromechanische Wandlereinheit (7), insbesondere ein piezoelektrisches Element, umfasst.

**11.** Inhalationstherapiegerät nach Anspruch 10, wobei die Oszillationserzeugungsvorrichtung (6, 7) eine Stützeinheit (6) umfasst, an der die elektromechanische Wandlereinheit (7) und die Membran (1) angebracht sind.

**12.** Inhalationstherapiegerät nach einem der Ansprüche 1 - 11, wobei die Verarbeitungseinheit (13) in die Steuereinheit (10) integriert ist.

**13.** Inhalationstherapiegerät nach einem der Ansprüche 1 - 12, wobei in der Steuereinheit (10) eine Energieversorgungseinheit für das Inhalationstherapiegerät integriert ist.

**Revendications**

1. Appareil de thérapie par inhalation présentant un générateur d'aérosol, comprenant

   - une membrane oscillante (1) pour nébuliser un liquide (3),
   - un dispositif (6, 7) de génération d'oscillation présentant au moins un élément de connexion (8, 9) pour fournir un signal d'activation et par lequel ladite membrane (1) est amenée à osciller lorsque le signal d'activation est fourni de telle manière qu'un liquide disposé sur un côté de ladite membrane est nébulisé à travers la membrane et est présent sur l'autre côté de ladite membrane sous forme d'aérosol,
   - une unité de commande (10), à partir de laquelle le signal d'activation peut être fourni à l'au moins un élément de connexion (8, 9) du dispositif (6, 7) de génération d'oscillation de sorte que ledit dispositif (6, 7) de génération d'oscillation amène la membrane (1) à osciller, et
   - une unité (13) de traitement configurée pour caler un signal de sortie causé par la respiration d'un patient au niveau de l'au moins un élément de connexion (8, 9) du dispositif (6, 7) de génération d'oscillation.

2. Appareil de thérapie par inhalation selon la revendication 1, dans lequel le signal de sortie calé est utilisé par l'unité de commande (10) pour commander le fonctionnement de l'appareil de thérapie par inhalation.

3. Appareil de thérapie par inhalation selon l'une des revendications 1 à 2, dans lequel l'unité (13) de traitement comprend une unité (21) de filtre passe-bas, une unité (22) de filtre passe-haut, et une unité (23) d'amplificateur.

4. Appareil de thérapie par inhalation selon l'une des revendications 1 à 2, dans lequel l'unité (13) de traitement comprend une unité (21) de filtre passe-bas, une unité (22) de filtre passe-haut et un convertisseur analogique-numérique pour convertir le signal de sortie calé.

5. Appareil de thérapie par inhalation selon la revendication 4, dans lequel le convertisseur analogique-numérique est connecté à une unité d'évaluation numérique pour évaluer le signal de sortie calé numérisé.

6. Appareil de thérapie par inhalation selon l'une quelconque des revendications 3 à 5, dans lequel l'unité (22) de filtre passe-haut filtre des composantes de fréquence inférieures à 0,1 Hz, de préférence inférieures à 0,05 Hz.

7. Appareil de thérapie par inhalation selon l'une des revendications 1 à 2, dans lequel l'unité (13) de traitement comprend une unité (21a) de filtre passe-bande et une unité (23) d'amplificateur, dans lequel l'unité (21a) de filtre passe-bande fournit une bande passante entre 0,1 Hz et au moins 10 Hz, de préférence entre 0,1 Hz et au moins 40 Hz.

8. Appareil de thérapie par inhalation selon l'une des revendications 1 à 2, dans lequel l'unité (13) de traitement comprend une unité (21b) de filtre passe-bas calée et une unité (23) d'amplificateur.

9. Appareil de thérapie par inhalation selon l'une des revendications 1 à 2, dans lequel l'unité (13) de traitement comprend une unité (21c) de filtre passe-bas calée, une unité (22c) de filtre passe-haut calée et une unité (23) d'amplificateur.

10. Appareil de thérapie par inhalation selon l'une des revendications 1 à 9, dans lequel le dispositif (6, 7) de génération d'oscillation comprend une unité (7) de transducteur électromécanique, en particulier un élément piézoélectrique.

11. Appareil de thérapie par inhalation selon la revendication 10, dans lequel le dispositif (6, 7) de génération d'oscillation comprend une unité (6) de support à laquelle l'unité (7) de transducteur électromécanique et la membrane (1) sont fixées.

12. Appareil de thérapie par inhalation selon l'une des revendications 1 à 11, dans lequel l'unité (13) de traitement est intégrée dans l'unité de commande (10).

13. Appareil de thérapie par inhalation selon l'une des revendications 1 à 12, dans lequel une unité d'alimentation en énergie pour l'appareil de thérapie par inhalation est intégrée dans l'unité de commande (10).

**Fig. 1**

EP 4 218 865 B1

# Fig. 2

# Fig. 3

Fig. 4

V(N008,N010)

Non-clamped respiration signal

Input signal

0.5s  1.0s  1.5s  2.0s  2.5s  3.0s  3.5s  4.0s  4.5s

V(N007,N010)

Clamped respiration signal

Input signal

0.5s  1.0s  1.5s  2.0s  2.5s  3.0s  3.5s

**Fig. 5**

**Fig. 6**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1304131 B1
- US 2019321570 A1
- EP 00973900
- EP 1227856 A
- US 6962151 B
- US 10129498 B
- EP 1558315 A1
- EP 15810730
- EP 3237047 A
- US 20170368282 A
- US 15535889 B